(19) Europäisches Patentamt

European Patent Office

Office européen des brevets

(11) EP 1 417 931 A1

(12) EUROPEAN PATENT APPLICATION

(43) Date of publication:
12.05.2004 Bulletin 2004/20

(51) Int Cl.⁷: **A61B 6/00**, A61B 19/00,
G01B 11/00, G01B 15/00

(21) Application number: 03078370.8

(22) Date of filing: 24.10.2003

(84) Designated Contracting States:
**AT BE BG CH CY CZ DE DK EE ES FI FR GB GR
HU IE IT LI LU MC NL PT RO SE SI SK TR**
Designated Extension States:
**AL LT LV MK**

(30) Priority: **05.11.2002 US 288012**

(71) Applicant: **EASTMAN KODAK COMPANY
(a New Jersey corporation)
Rochester, New York 14650 (US)**

(72) Inventors:
• **Foos, David H., Eastman Kodak Company
Rochester New York 14650-2201 (US)**
• **Wang, Xiaohui, Eastman Kodak Company
Rochester New York 14650-2201 (US)**

(74) Representative: **Weber, Etienne Nicolas et al
Kodak Industrie,
Département Brevets,
CRT,
Zone Industrielle
71102 Chalon sur Saône Cedex (FR)**

(54) **Method for automatically producing true size radiographic image**

(57)    A reference system (10) for use in determining the degree of magnification of a radiographic image comprising: a reference object (12) of predetermined dimensions constructed of radiation attenuating material; a flexible elongated member (14) for mounting the reference object; and adjustable means (16) associated with the elongated member for holding the elongated member in place when the elongated member is wrapped around an anatomical part to be radiographically imaged (201). A method for producing a radiographic image that represents anatomical features of an individual at approximately true size comprising: positioning a reference object (12) of predetermined dimensions and of radiation attenuating material in the radiographic imaging path at approximately the same distance from a radiographic imaging receptor (301) in a location that is adjacent to the individual's anatomy that is being radiographically imaged (201); producing a digital radiographic image (401) of the individual's anatomy and reference object; measuring the size of the digitized reference object and calculating a scale factor as a function of the measured size and the known predetermined size of the reference object (12); and associating the scale factor with the digital radiographic image so that it can be scaled to true anatomical size.

FIG. 2

EP 1 417 931 A1

Description

FIELD OF THE INVENTION

This invention relates to digital radiography, and more particularly to a method for producing a radiographic image that represents anatomical features of an individual at approximately true size and to a reference system for use in determining the degree of magnification of a radiographic image.

BACKGROUND OF THE INVENTION

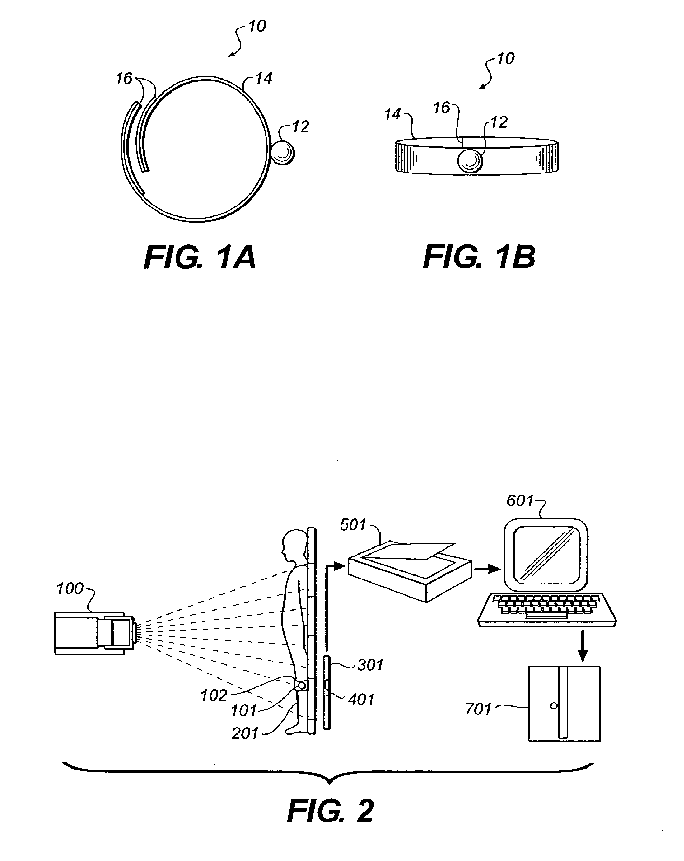

Orthopedic surgeons make a variety of measurements from radiographic images as part of the surgical planning process. These include both angular and absolute distance measurements. It is important that these measurements are accurate and precise because the sizes of prosthetic implants are determined based on these measurements. The accuracy and precision of measurements are also important for non-orthopedic applications, such as oncology where the size of a lesion is tracked over time to make assessments of malignancy based on doubling times, or to determine the efficacy of a treatment in reducing the size of a tumor. An important element of the image chain that affects the accuracy of absolute distance measurements made from x-ray images is magnification. Magnification is introduced by the increase in optical path length from the patient anatomy to the imaging receptor of the incident x-ray light originating from a point source. The imaging receptor may be a cassette containing a screen-film imaging system, a cassette containing a storage phosphor screen for computed radiography (CR), or a flat panel digital radiography detector (DR). Figure 1 illustrates how the image of the anatomy is magnified with respect to the true size of the object that is imaged. The degree of magnification is directly related to the distance between the anatomy being imaged and the imaging receptor. This distance will vary if different x-ray tables are used, wherein the distance between the tabletop and the placement of the receptor within the table is a nonstandard distance. This distance will also vary depending upon the body part that is imaged, positioning of the body part, and patient thickness.

X-ray tables for analog screen film systems are standardized such that distance from tabletop to receptor is fixed and known. Measurements from traditional screen-film captured images are then made using rulers that have fixed levels of magnification factors built in to approximately account for different patient thickness and different body parts. Digital x-ray capture devices have introduced a new element of variability in tabletop to receptor distance. Moreover, when digital capture is coupled with the ability to make digital measurements using workstations, an increased degree of accuracy and precision in the overall process for making these measurements is expected. Placement of a calibration device (object of known dimensions) in the imaging path can be used as a means to calculate a magnification factor to achieve improved accuracy. U.S. Patent 6,459,772, filed Mar. 17, 2000, inventors, Wiedenhoefer et al., discloses a method for accurately calculating the degree of magnification of radiographic images based on a device comprised of multiple parts including radio-opaque sphere, attenuation plates, a radiolucent structure for housing the reference sphere, and an adhesive material used to affix the cubicle housing to the patient. While this device provides a means for obtaining an accurate magnification factor, the device is cumbersome and not well designed for easy use by radiographic technologists in clinical practice with patients. The cubicle design of the housing may be difficult to position for certain radiographic projections and views. The design is further complicated because there is both a disposable as well as a reusable portion. Moreover, the method is incomplete because it does not include a method to automatically measure the magnification factor or to then resize a digital radiograph to true size based on the measured data.

It is therefore desirable to provide a practical, fully reusable method for making measurements of anatomy in clinical practice from digital radiographic captured images (CR and DR) and to provide an automated means for providing a resultant true size image for diagnostic interpretation and surgical planning.

SUMMARY OF THE INVENTION

According to the present invention, there is provided a solution to the problems discussed above.

According to a feature of the present invention, there is provided a reference system for use in determining the degree of magnification of a radiographic image comprising: a reference object of predetermined dimensions constructed of radiation attenuating material; a flexible elongated member for mounting said reference object; and adjustable means associated with said elongated member for holding said elongated member in place when said elongated member is wrapped around an anatomical part to be radiographically imaged.

ADVANTAGEOUS EFFECT OF THE INVENTION

The invention has the following advantages.

1. The method is extremely convenient for radiologists, orthopedic surgeons, and radiographic technologist to use in clinical practice.
2. The use of an adjustable size Velcro strap facilitates the use of the method with various patient anatomies in clinical practice and a minimal number of steps are required by the technologists to affix the device. The strap facilitates the adjustment of the height of the spherical reference marker to match the height of the region of interest with respect to the image receptor and also positions the reference marker immediately adjacent to the region of interest thereby minimizing positioning errors. The device is completely reusable.

3. The method automatically rescales the captured digital image to a true anatomical size, thereby removing the need for radiologists and orthopedic surgeons to assume correction factors for magnification and allowing direct use of measured data made using standard measurement tools provided on diagnostic and clinical workstations.

4. This method automatically compensates for magnification differences that may be encountered in clinical practice due to different distances between patient and receptor if different x-ray tables are used, and more accurately accounts for magnification differences that are encountered among patients caused by differences in patient thickness.

5. The use of this method ensures that regardless of which x-ray table is used, and regardless of patient thickness, measurements made of anatomical features from printed radiographic images or images displayed on a diagnostic or clinical workstations, will have improved accuracy.

## BRIEF DESCRIPTION OF THE DRAWINGS

Figs. 1A and 1B are diagrammatic views showing one embodiment of the invention comprised of a spherical reference object made from x-ray attenuating material that is fastened to a Velcro strap.

Fig. 2 is a diagrammatic view showing the general setup for x-ray imaging and cause of magnification consisting of patient, x-ray source, x-ray table or upright Bucky mechanism, and imaging receptor (screen film system in cassette, storage phosphor screen in cassette, or flat panel digital x-ray detection).

Figs. 3 and 4 are diagrammatic views useful in explaining the present invention.

## DETAILED DESCRIPTION OF THE INVENTION

According to a feature of the present invention, there is provided a method for automatically producing a radiographic image of anatomical features such that the features are represented in the image at approximately true size. The method utilizes a reference object of known size that is constructed of x-ray attenuating material. In the preferred embodiment of the invention the reference object will be a sphere. In the preferred embodiment of the invention the reference object is fastened to a Velcro strap that is made of radio-transparent material. A radiographic technologist is then able to conveniently position the reference object on the patient by wrapping the strap around the anatomy that is being imaged. The strap is adjusted such that reference object is positioned in the x-ray imaging path at approximately the same distance from the imaging receptor in a location that is adjacent to the patient anatomy that is being imaged. A magnification factor is calculated from the image by automatically detecting the reference object in software, automatically measuring the size of the image of the reference object, and comparing the known actual size of the reference object against the size of the reference object that is measured from the image data. The magnification factor is then automatically converted to a scale factor for resizing (minifying) the image such that the pixel spacing in the minified image represents true anatomical size. The image of the anatomy is then either printed or displayed on a workstation at true anatomical size. The scale factor can also be provided in digital form as part of the image header at the capture device so that it can be transmitted to a diagnostic workstation where the image can be scaled to represent true anatomical size

In Figs. 1A and 1B there is illustrated one embodiment of the invention comprising: a reference system 10 for use in determining the degree of magnification of a radiographic image. System 10 includes a reference object 12 of predetermined dimensions constructed of radiation attenuating material such as lead, brass or aluminum. Object 12 is preferably substantially spherical in shape having a predetermined diameter. System 10 also includes a flexible elongated member 14, such as a belt or the like for mounting reference object 10. Adjustable means such as Velcro strips 16 on member 14 are used to hold member 14 in place when it is wrapped around an anatomical part of an individual which is to be radiographically imaged. Other adjustment means include other type of fasteners such as buckles, snaps, etc. Member 14 can also form a continuous band having elastic segments for adjustable positioning on an individuals arm, leg or torso. Object 12 can be mounted on member 14 by any means such as Velcro patches, grommets, or the like.

Another embodiment of the invention is shown in Fig. 2. The x-ray attenuating reference object 101 is fastened to the radio-transparent strap 102. The radio-transparent strap 102 is fitted to the patient anatomy of interest 201 (in this example the femur). The strap 102 is adjusted such that the reference object 101 is aligned adjacent to the anatomy of interest 201 and at the same height above the image receptor 301 as the anatomy being radiographically imaged.

The x-ray attenuating reference object 101 is fastened to the radio-transparent strap 102. The radio-transparent strap 102 is fitted to the patient anatomy of interest 201 (in this example the femur). The strap 102 is adjusted such that the reference object 101 is aligned adjacent to the anatomy of interest 201 and at the same height above the image receptor 301 as the anatomy of interest 201. An x-ray exposure from x-ray source 100 of the patient anatomy of interest 201 is then performed. A radiographic image 401 is captured by the imaging receptor 301 of the patient anatomy of interest 201 and the x-ray attenuating reference object 101. If imaging receptor 301 is x-ray film or a storage phosphor imaging member, a digital version of the image 401 is produced using a scanner 501. If imaging receptor 301 is a direct digital image is produced and scanner is not needed. In any case, the digital image is then analyzed on a computer 601 to automatically detect and measure the size of the image of the reference object 401 and compare this measured size against the actual size of the reference object 101. The ratio of the actual size of the reference object 101 to the measured size of the reference object is used as a scale factor by the computer 501 to resize (minify) the image 401 to create a new image 701 such that the pixel spacing in the minified image represents true anatomical size. (Alternatively, the original image and scale size in a header can be sent together to an output device where the original image is resized). Image 701 can be displayed on an electronic display or output as a hardcopy media (film). The location and size of the reference sphere are detected automatically once the image is acquired in digital format.

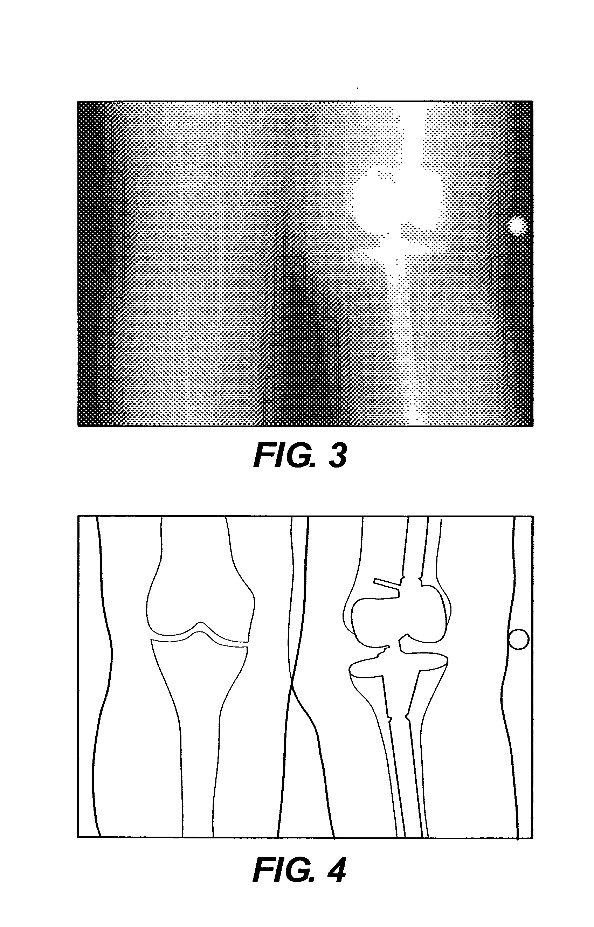

As shown in Figure 3, the sphere appears in the image as a circular object. There are many methods that can be used to detect such a regular shape. One preferred embodiment of finding the circular object in the image is using a Hough transform. (See: V. Hough, "Method and means for recognizing complex patterns", U.S. Patent 3,069,654, filed Mar. 25, 1960. J. Illingworth, "A survey of the Hough transform," Computer Vision Graphics and Image Processing, vol. 44, pp. 87-116, 1988. V. Leavers, "Which Hough transform ?", Computer Vision Graphics and Image Processing: Image Understanding, vol. 58, no. 2, Sept, pp. 250-264, 1993, C. Kimme, D. Ballard, and J. Slansky, "Finding circles by an array of accumulates", Communication of the ACM, vol. 18, no. 2, pp. 120-122, 1975 of the edge information Fig. 4).) To speed the computation, one can use a multi-resolution approach of the Hough transform. (See: M. Atiquzzaman, "Multi-resolution Hough Transform - an efficient method of detecting pattern in images", IEEE transactions on Pattern Analysis and Machine Ittelligence, 14(11), pp. 1090-1095, Nov. 1992., the Fast Hough transform H. Li, M. A. Lavin and R. J. LeMaster, "Fast Hough transform", Computer Vision Graphics and Image Processing, vol. 36, pp 139-161, 1986., or adaptive Hough transform J. Illingworth and J. Kittler, "Adaptive Hough transform", IEEE transactions on Pattern Analysis and Machine Ittelligence, 9(5), pp 690-698, Sept. 1987.)

After the size of the circular object is estimated, the magnification scale factor can be calculated as:

$$Mag = D_{measured} \text{ in pixels x PixelSize} / D_{actual} \text{ size,}$$

Where $D_{measured}$ in pixels is the circle diameter in pixels, and $D_{actual \, size}$ is the actual size of the reference sphere. Either the image itself can be minified based on the magnification factor while preserving the original pixel size unchanged, or the image itself is kept the same but the actual pixel size is magnified accordingly.

The invention has been described in detail with particular reference to certain preferred embodiments thereof, but it will be understood that variations and modifications can be effected within the spirit and scope of the invention.

**Claims**

1. A reference system for use in determining the degree of magnification of a radiographic image comprising:

   a reference object of predetermined dimensions constructed of radiation attenuating material;
   a flexible elongated member for mounting said reference object; and
   adjustable means associated with said elongated member for holding said elongated member in place when said elongated member is wrapped around an anatomical part to be radiographically imaged.

2. The reference system of claim 1 wherein said reference object is substantially spherical and has a predetermined diameter.

3. The reference system of claim 1 wherein said flexible elongated member is an elongated strip having first and second ends and wherein said adjustable means includes complementary Velcro strips on said elongated strip that allow said first and second ends of said strip to be adjustably affixed to each other after said strip is positioned on said anatomical part.

4. A method for producing a radiographic image that represents anatomical features of an individual at approximately true size comprising:

   positioning a reference object of predetermined dimensions and of radiation attenuating material in the radiographic imaging path at approximately the same distance from a radiographic imaging receptor in a location that is adjacent to the individual's anatomy that is being radiographically imaged;
   producing a digital radiographic image of the individual's anatomy and reference object;
   measuring the size of the digitized reference object and calculating a scale factor as a function of said measured size and the known predetermined size of said reference object; and associating said scale factor with said digital radiographic image so that it can be scaled to true anatomical size.

5. The method of claim 4 wherein in positioning said reference object said reference object is a sphere of predetermined size mounted on an elongated member that is wrapped around the anatomy of said individual in the radiographic imaging path at approximately the same distance from a radiographic imaging receptor in a location that is adjacent to the individual's anatomy being radiographically imaged.

6. The method of claim 4 wherein said producing a digital radiographic image includes producing a radiographic image in imaging media and scanning said imaging media to produce said digital radiographic image.

7. The method of claim 4 wherein said producing a digital radiographic image includes producing a radiographic image in a storage phosphor imaging member and scanning said storage phosphor imaging member to produce said digital radiographic image.

8. The method of claim 4 wherein said producing a digital radiographic image includes producing a radiographic image in a digital radiographic imaging device.

9. The method of claim 4 including using said scale factor to scale said digital radiographic image to an output digital radiographic image having a true anatomical size.

10. The method of claim 9 including presenting said output digital radiographic image in a true anatomical size on an electronic display.

11. The method of claim 9 including presenting said output digital radiographic image in a true anatomical size in hardcopy media.

**FIG. 1A**

**FIG. 1B**

**FIG. 2**

**FIG. 3**

**FIG. 4**

**European Patent Office**

## EUROPEAN SEARCH REPORT

Application Number

EP 03 07 8370

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | WO 99 59106 A (HATCHER DAVID C ;PALM CHARLES (US); ACUSCAPE INTERNATIONAL INC (US) 18 November 1999 (1999-11-18)<br>* page 12, line 6-8 *<br>* page 14, line 7-9 *<br>* page 17, line 1 - page 19, line 15 *<br>* page 21, line 13-19 *<br>* page 25, line 3-6 *<br>* page 28, line 12 - page 29, line 8 *<br>* page 101, line 12-15 *<br>* figures 1,4,7-20,26-31,41 *<br>--- | 1-11 | A61B6/00<br>A61B19/00<br>G01B11/00<br>G01B15/00 |
| A | US 5 848 125 A (ARNETT G WILLIAM) 8 December 1998 (1998-12-08)<br>* abstract; figures 1-3,5,6 *<br>* column 3, line 20 - column 4, line 18 *<br>--- | 1,2,4,5 | |
| A | GB 1 458 196 A (LOWNDES R B W) 8 December 1976 (1976-12-08)<br>* page 1, line 12-39 *<br>* page 1, line 89-91 *<br>* page 2, line 39-43 *<br>----- | 1,4 | TECHNICAL FIELDS SEARCHED (Int.Cl.7)<br><br>A61B<br>G01B |

The present search report has been drawn up for all claims

| Place of search | Date of completion of the search | Examiner |
|---|---|---|
| BERLIN | 24 February 2004 | Popovici, M |

EPO FORM 1503 03.82 (P04C01)

## ANNEX TO THE EUROPEAN SEARCH REPORT
## ON EUROPEAN PATENT APPLICATION NO.

EP 03 07 8370

This annex lists the patent family members relating to the patent documents cited in the above-mentioned European search report.
The members are as contained in the European Patent Office EDP file on
The European Patent Office is in no way liable for these particulars which are merely given for the purpose of information.

24-02-2004

| Patent document cited in search report | | Publication date | Patent family member(s) | | Publication date |
|---|---|---|---|---|---|
| WO 9959106 | A | 18-11-1999 | AU<br>EP<br>WO | 4076999 A<br>1027681 A1<br>9959106 A1 | 29-11-1999<br>16-08-2000<br>18-11-1999 |
| US 5848125 | A | 08-12-1998 | JP | 11169363 A | 29-06-1999 |
| GB 1458196 | A | 08-12-1976 | NONE | | |

For more details about this annex : see Official Journal of the European Patent Office, No. 12/82